# FASCICULE DE BREVET EUROPEEN

(11) **EP 0 414 633 B1**
(45) Date de publication et mention de la délivrance du brevet: **26.07.1995**
(21) Numéro de dépôt: 90810599.2
(22) Date de dépôt: 08.08.1990
(51) Int. Cl.: A61B 17/60

(54) **Fixateur externe dynamique pour poignet**
Dynamische Aussenbefestigung für das Handgelenk
Dynamic external fixation device for the wrist

(30) Priorité: 23.08.1989 CH 3060/89
(43) Date de publication de la demande: 27.02.1991
(73) Titulaire: JAQUET ORTHOPEDIE S.A., CH-1228 Plan-les-Ouates (CH)
(72) Inventeur: Asche,Gernot, D-7290 Freudenstadt (DE); Wagenknecht,Marcel, CH-1219 Le Lignon (CH)
(74) Mandataire: Dietlin, Henri

(56) Documents cités:
- EP-A- 0 192 440
- EP-A- 0 248 138
- EP-A- 0 256 984
- GB-A- 2 110 094

## Description

La présente invention est du domaine des équipements médicaux et concerne plus particulièrement un fixateur externe dynamique pour poignet.

Depuis plusieurs années, on sait que l'immobilisation d'un os fracturé au voisinage d'une articulation a pour effet secondaire un engourdissement de l'articulation qui nécessite souvent une longue rééducation. On a donc développé une technique utilisant des fiches insérées dans l'os, de part et d'autre de la fracture, qui sont reliées par un fixateur comportant un joint articulé autorisant, sous contrôle médical, un mouvement naturel de l'articulation.

Par exemple lors de fractures dans la partie distale du radius, il faut que le fixateur externe puisse autoriser un mouvement naturel du poignet, mouvement dont l'amplitude peut être augmentée au cours de la consolidation osseuse. Dans ce cas, les fiches sont insérées dans le radius d'une part et dans le métacarpe d'autre part.

Il est nécessaire que le fixateur suive le mouvement naturel de l'articulation et il doit donc pouvoir être ajusté par rapport au mouvement anatomique du poignet, pour éviter toute dislocation de la fracture.

On connaît déjà par le brevet US-4.628.919 (Clyburn) un fixateur destiné à être porté sur le côté intérieur de l'avant-bras et comportant une branche postérieure et une branche antérieure reliées par un joint à rotule, chaque branche servant à la fixation des fiches. La branche antérieure permet en outre d'appliquer une élongation ou contraction contrôlée. De plus, le joint à rotule comporte des moyens de réglage du degré de mouvement relatif entre les branches postérieure et antérieure.

Au cours de la consolidation, on a constaté qu'il est préférable de limiter le mouvement du poignet à un mouvement de flexion de la main par rapport à l'avant-bras plutôt que d'autoriser un mouvement omnidirectionnel de la main.

A cet effet la déposante a déjà proposé, dans le brevet européen ... (publication No 0.248.138), un fixateur externe dynamique pour l'ostéosynthèse d'une articulation fracturée comprenant un premier membre ayant une tige de fixation apte à être reliée à des fiches insérées dans l'os d'un côté de l'articulation; un second membre ayant une tige de fixation apte à être reliée à des fiches insérées dans un os de l'autre côté de l'articulation; et un joint articulé reliant ces deux membres qui possède une surface de glissement courbe possédant un axe virtuel, ce joint articulé comportant des moyens de visée de l'axe de la surface de glissement.

On a constaté qu'il n'est pas suffisant de faire coïncider l'axe de la surface de glissement avec le centre de l'articulation et qu'il est nécessaire de pouvoir en outre incliner transversalement le joint articulé par rapport à l'axe longitudinal de l'appareil, sans toutefois dérégler ce dernier, afin de pouvoir adapter plus précisément le fixateur à l'anatomie du patient.

La présente invention a en conséquence pour objet un fixateur externe dynamique pour l'ostéosynthèse d'une articulation fracturée comprenant :
a) un premier membre ayant une tige de fixation apte à être reliée à des fiches insérées dans l'os d'un côté de l'articulation;
b) un second membre ayant une tige de fixation apte à être reliée à des fiches insérées dans un os de l'autre côté de l'articulation;
c) et un joint articulé reliant les tiges qui possède :
   - un boîtier (11) présentant une surface de glissement courbe possédant un axe virtuel,
   - des moyens de visée dudit axe, et
   - un segment arqué apte à glisser sur ladite surface.

Ce fixateur est caractérisé par le fait que les dites tiges sont aptes à pivoter sur leur axe par rapport au boîtier pour autoriser le positionnement transversal du joint articulé sans affecter le réglage longitudinal.

Dans une variante préférentielle, le fixateur externe comporte en outre un dispositif autorisant de l'ébat à l'une des tiges de fixation par rapport au joint articulé.

Les dessins annexés représentent, à titre d'exemples non limitatifs, quelques formes d'exécution de l'objet de la présente invention.

La figure 1 est une vue latérale d'un poignet sur lequel est disposé un fixateur externe dynamique selon l'invention.

La figure 2 est une vue de dessus de l'ensemble de la figure 1.

La figure 3 est une vue latérale du dispositif de la figure 1, depuis la face opposée, montrant l'amplitude maximum du mouvement autorisé.

La figure 4 est une coupe longitudinale des éléments de la figure 3.

La figure 5 est une coupe transversale selon V-V à la figure 4, montrant le blocage de l'ajustement angulaire de la partie antérieure.

La figure 6 est une coupe transversale selon VI-VI à la figure 4, montrant les pièces constituant l'ajustement angulaire de la partie postérieure.

La figure 7 est une coupe transversale selon VII-VII à la figure 4, à une échelle plus grande, montrant les éléments de guidage d'une coulisse arquée.

La figure 8 représente en détail les pièces constituant l'ajustement angulaire de la partie antérieure, vues de dessus dans la moitié inférieure du dessin et en coupe dans la partie supérieure.

La figure 9 est une vue similaire à celle de la figure 8, les pièces étant vues latéralement dans la moitié inférieure du dessin et en coupe dans la partie supérieure.

La figure 10 est une coupe transversale selon X-X à la figure 9.

La figure 11 est une vue latérale d'un dispositif amovible de guidage des aiguilles de visée.

La figure 12 est une coupe transversale selon XII-XII à la figure 11.

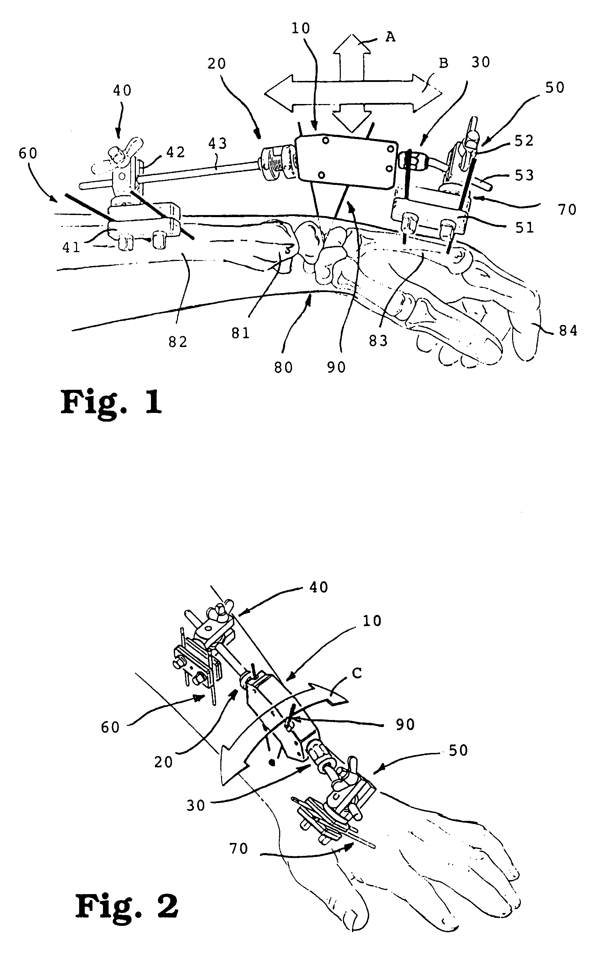

Dans la représentation des figures 1 et 2, le fixateur externe dynamique selon l'invention est constitué par un joint articulé 10 disposé entre un dispositif de réglage 20 et un dispositif autorisant de l'ébat 30.

Les dispositifs 20 et 30 sont respectivement solidaires d'un ensemble postérieur 40 et d'un ensemble antérieur 50 de fixation de fiches 60 et 70 insérées dans les os de part et d'autre d'une articulation 80. Des aiguilles 90, solidaires du joint articulé 10, permettent de viser le point central de l'articulation 80.

Les ensembles postérieur et antérieur 40 et 50 sont constitués, de manière conventionnelle, d'un portefiches 41, respectivement 51, orientable par rapport à une bride 42, 52 de positionnement et de serrage sur une tige de fixation 43, 53, solidaire respectivement des dispositifs 20 et 30.

Dans le cas d'une fracture de la tête 81 du radius 82 du poignet 80, les groupes de fiches 60 et 70 sont insérées dans le radius 82 et dans le métacarpe 83 de l'index 84. Les fixateurs 40 et 50 sont montés sur les fiches et les tiges de fixation 43 et 53, et le joint articulé 10 est positionné, grâce aux aiguilles 90 permettant de viser l'axe de l'articulation 80. C'est en agissant sur les ensembles 40 et 50, que le joint articulé 10 peut être déplacé selon les flèches A et B de la figure 1.

En se référant à la figure 2, on voit que le joint 10 peut également être positionné angulairement selon la flèche C, c'est-à-dire transversalement par rapport à l'axe longitudinal de l'appareil.

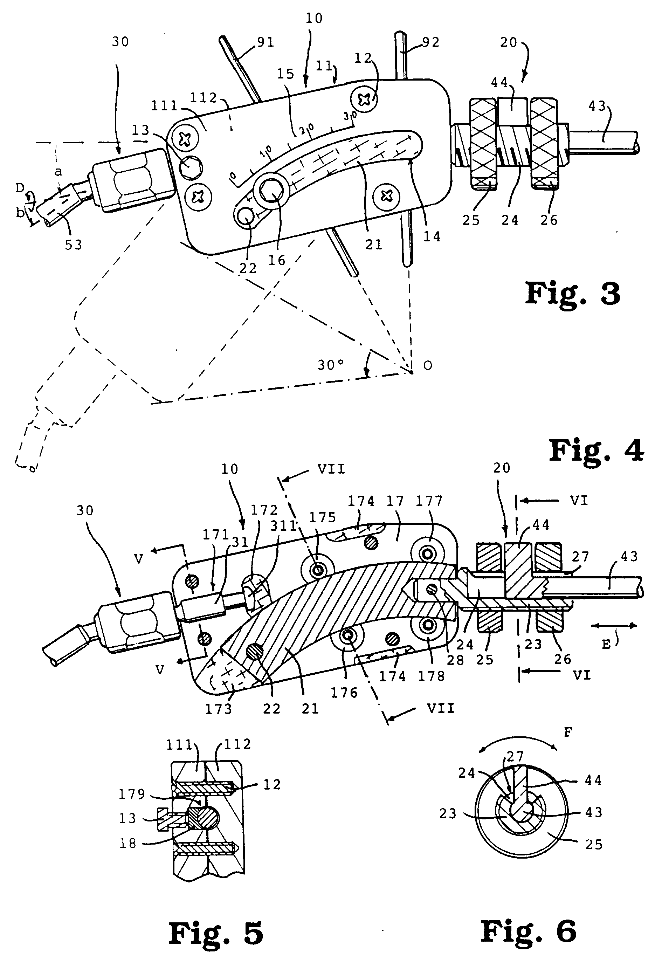

A la figure 3, le joint articulé 10 est représenté vu latéralement. Il est constitué par un boîtier 11 en deux parties 111 et 112, serrées l'une contre l'autre au moyen de vis 12, à tête noyée dans le boîtier. Une vis 13 permet de bloquer angulairement le dispositif 30 autorisant un ébat de l'ensemble après l'avoir positionné par rapport au joint articulé 10 selon la flèche D. La moitié 111 du boîtier comporte une ouverture arquée 14, centrée sur l'axe O de l'articulation, ainsi qu'une échelle 15 graduée en degrés. L'ouverture 14 comporte une vis de butée 16, solidaire d'un cavalier mobile qui sera décrit plus loin. On remarquera que les vis 13 et 16 ont toutes la même tête à 6 pans, pour que les réglages puissent être effectués avec un outil unique.

Dans l'ouverture arquée 14 apparaît la coulisse arquée 21, qui est solidaire du dispositif 20 et qui porte un piton transversal 22, destiné à buter d'une part contre le bord antérieur de l'ouverture 14 et d'autre part contre le cavalier mobile solidaire de la vis de butée 16.

La moitié 112 du boîtier comporte en outre deux passages pour les aiguilles de visée 91 et 92 utilisées pour centrer l'ensemble sur l'axe O de l'articulation, comme on le verra par la suite.

A la figure 3 on a représenté le joint articulé dans sa position de repos en traits pleins et dans sa position de flexion maximum en traitillés. On notera que dans la position de repos figurant en traits pleins, la tige 43 fait un angle a d'environ 10° par rapport à l'axe du dispositif 20 afin de suivre au mieux la configuration de la position de repos du poignet. Selon l'anatomie propre à chaque individu, on peut encore prévoir une tige de fixation 53 faisant un coude d'un angle b pouvant atteindre environ 20°.

Le dispositif de réglage transversal 20 est constitué par un corps cylindrique fileté 23 disposé en bout de la coulisse arquée 21, qui présente extérieurement une ouverture centrale 24. La tige de fixation postérieure 43 peut être déplacée axialement dans l'ouverture centrale 24 du corps cylindrique 23 selon la flèche E, à l'aide des écrous molletés 25 et 26 disposés de part et d'autre de l'ergot 44 de l'extrémité de la tige 43.

Dans la coupe de la figure 4 du dispositif 20 on remarque que l'ouverture centrale 24 est prolongée vers le haut par un dégagement latéral 27 pour permettre l'orientation selon la flèche F à la figure 6 de l'ergot 44. Dans la variante proposée au dessin, la liaison entre la coulisse arquée 21 et le corps cylindrique 23 est réalisée au moyen d'une goupille 28.

La face intérieure 17 de chaque moitié 111 et 112 du boîtier 11 comporte une série de dégagements, à savoir :
- un dégagement cylindrique 171 pour la tige intérieure 31 du dispositif 30 autorisant un ébat de l'ensemble;
- une creusure 172 pour la tête 311 de la tige 31, afin de maintenir le dispositif 30 dans le boîtier 10:
- un dégagement arqué 173 pour permettre le passage de la coulisse arquée 21;
- deux dégagements plans 174, prévus sur des faces opposées, dont l'utilité sera mentionnée plus loin; ainsi que
- quatre ouvertures 175 à 178 destinées à recevoir des roulements 19 favorisant le glissement de la coulisse 21.

L'une des moitiés 111 ou 112 du boîtier comporte en outre une ouverture taraudée s'ouvrant dans un dégagement 179, visible à la figure 5, dans laquelle la vis 13 permettant de positionner angulairement le dispositif 30 est vissée. La vis 13 coopère avec un sabot 18 disposé dans le dégagement 179. L'une des faces du sabot 18 comporte un dégagement cylindrique de dimension correspondant à la tige intérieure 31.

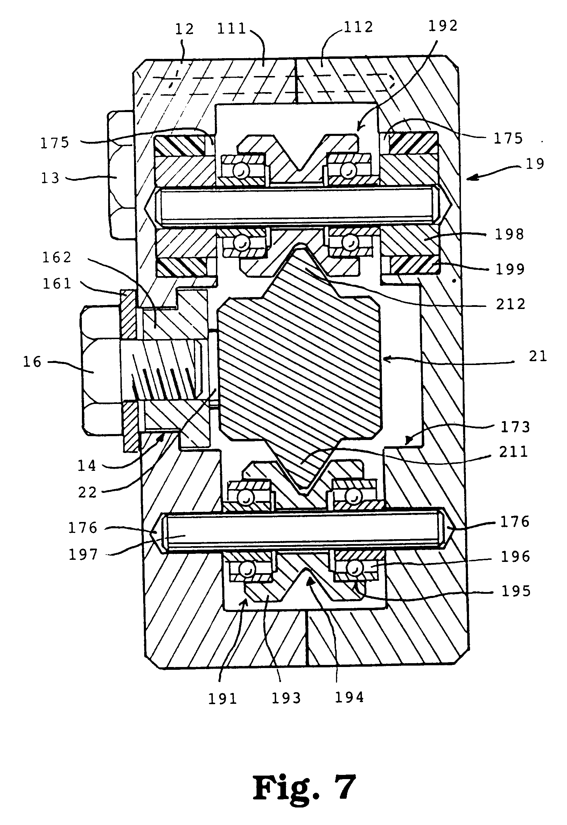

Dans la coupe de la figure 7 on retrouve les moitiés 111 et 112 constituant le boîtier 11 du joint articulé, et l'une des vis 12 de liaison. On voit également la vis 13 de positionnement angulaire et la vis de butée 16, dont la tête appuie sur une rondelle 161 et qui est solidaire d'un cavalier mobile 162 disposé à l'intérieur de l'ouverture arquée 14. Le cavalier mobile 162 sert de butée au piton transversal 22 de la coulisse arquée 21.

On notera la forme particulière de la section de la coulisse arquée 21 qui comporte deux épaulements 211 et 212, de section cônique, destinés à coopérer avec des ensembles de roulement 19. On notera que les ensembles de roulement 191 destinés à coopérer avec l'épaulement arqué 211 différent des ensembles de roulement 192 destinés à coopérer avec l'épaulement arqué 212.

Chaque ensemble de roulement 191 comporte un galet 193 comportant une gorge 194 de forme correspondante à l'épaulement arqué 211. Les faces du galet 193 comportent des dégagements 195 destinés à recevoir des roulements à billes 196 solidaires d'un arbre central 197.

L'ensemble de roulement 192 comporte en outre des paliers 198, disposés dans les ouvertures 175 et 177 pratiquées dans les parois 111 et 112. Les paliers 198 sont entourés par un joint élastomère 199. En variante, on peut remplacer ce joint par une série de O-rings. L'introduction d'une matière élastomère a pour fonction d'établir une légère pré-contrainte permettant d'éliminer les jeux.

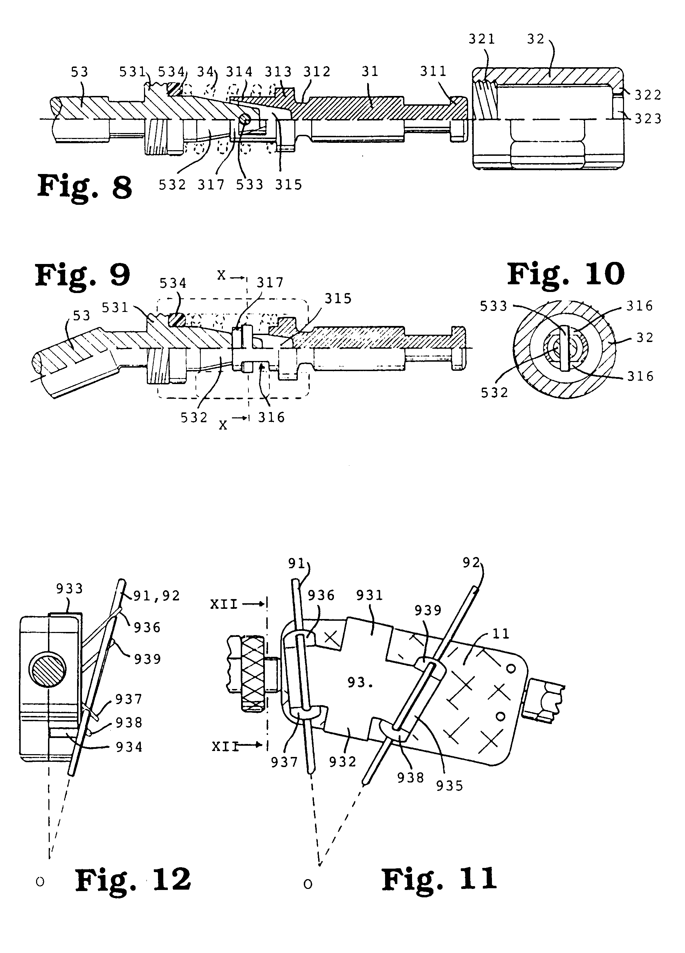

En se référant aux figures 8 à 10 représentant en détail les pièces constituant le dispositif 30 autorisant de l'ébat, on retrouve la tige intérieure 31 et sa tête 311 destinée à maintenir l'ensemble par rapport au boîtier 11. Vers l'extérieur, la tige 31 comporte en outre une creusure 312, un épaulement 313 et se termine par une partie cylindrique 314. Cette partie 314 comporte une ouverture centrale troncônique 315 et deux dégagements latéraux opposés 316 (visibles aux figures 9 et 10), constituant une collerette de retenue 317 destinée à assurer la liaison avec la tige de fixation 53.

La tige de fixation 53 comporte un épaulement fileté 531 et se termine par une terminaison tronconique 532, de forme correspondant à celle de l'ouverture centrale 315 de la tige intérieure 31. La terminaison 532 reçoit une goupille 533 de retenue. Un ressort 34 (représenté partiellement en traitillés au dessin) est disposé entre les épaulements 531 et 313 et est destiné à écarter les tiges 31 et 53 l'une de l'autre.

Un manchon 32 est destiné à recouvrir l'ensemble décrit et comporte à une extrémité un taraudage 321 destiné à coopérer avec l'épaulement fileté 531, et à l'autre extrémité un rebord interne 322 destiné à coopérer avec l'épaulement 313, pour assurer la compression du ressort 34, ce rebord délimitant une ouverture centrale 323 assurant le passage de la tige intérieure 31. On ajoutera avantageusement un joint en silicone 534 en prolongement de l'épaulement 531, qui assure une fonction de frein pour éviter le desserrement intempestif du manchon 32.

Aux figures 11 et 12, on a représenté une variante pour positionner les aiguilles de visée 91 et 92, au moyen d'une plaquette ressort 93. La plaquette 93 comporte deux branches 931 et 932, repliées deux fois de 90° vers l'intérieur de manière que leur côté 933 et 934 épouse la forme de la moitié du boîtier 11 et que leur extrémité vienne s'engager dans les deux dégagements plans 174 décrits en regard de la figure 4. La plaquette 93 comporte deux bras latéraux 935 dont les bords opposés comportent des languettes obliques 936 à 939 présentant une ouverture pour le passage des aiguilles 91 et 92.

En raison de l'élasticité de la plaquette 93, il est possible de la mettre en place facilement sur le boîtier 11, par déformation des côtés 933 et 934 au cours de l'introduction des griffes terminales des branches 931 et 932 dans les dégagements 174. Les languettes obliques 936 et 937 sont serrées l'une vers l'autre pour autoriser la mise en place de l'aiguille de visée 91 de même que l'on presse les languettes 938 et 939 pour passer l'aiguille 92.

La majorité des constituants du fixateur décrit est réalisée en acier inoxydable, seul le boîtier 10 et la coulisse arquée étant en alliage léger, afin de diminuer le poids de l'ensemble.

Avant d'être mis à la disposition du praticien, le dispositif est monté comme représenté au dessin.

A cet effet, le segment arqué 21 est muni de sa butée 22 et est solidarisé par la goupille 27 du corps cylindrique fileté 23 qui reçoit la tige de fixation 43, fixée par les écrous molletés 25 et 26.

La tige intérieure 31 du dispositif 30 et la tige de fixation 53 sont associées par insertion de la goupille de retenue 533, après mise en place du ressort 34. Puis le manchon 32 est placé sur l'ensemble.

On remarquera que tant que le manchon n'est pas totalement serré, la tige 31 du dispositif 30 autorisant de l'ébat et la tige de fixation 53 sont articulées l'une par rapport à l'autre. En effet la terminaison tronconique 532 n'est que partiellement engagée dans l'ouverture tronconique 315, sous l'effet du ressort 34 qui écarte les épaulements 531 et 313 l'un de l'autre. Les tiges 31 et 53 restent toutefois toujours reliées, car la goupille de retenue 533 vient buter en position d'écartement extrême contre la collerette 317.

Les extrémités de la goupille 533 peuvent se déplacer librement dans les dégagements latéraux opposés 316 de la tige 31, et le mouvement résultant du manchon 32 par rapport à la tige 31 est rendu possible puisque son rebord interne 322 peut être déplacé dans la creuçure 312. On notera encore qu'en plus de l'articulation relative des tiges 31 et 53, la construction proposée autorise un léger pivotement de la tige 53 par rapport à la tige 31, pivotement qui est limité dès que les extrémités de la goupille de retenue 533 butent sur les bords des dégagements 316. Au fur et à mesure que le manchon 32 est vissé sur l'épaulement fileté 531, le mouvement relatif entre les tiges 31 et 53 diminue.

Comme on l'a déjà mentionné, le joint en silicone 534 sert de frein au mouvement du manchon 32, pour éviter son déplacement intempestif.

Pour terminer le montage du fixateur selon l'invention, les dispositifs 20 et 30 sont mis en place dans le boîtier 11. Le segment arqué 21 est disposé entre les ensembles de roulement 19 de manière que la butée 22 fasse saillie , dans l'ouverture 14, entre la vis 16 et la partie antérieure de l'ouverture. La tête 311 de retenue de la tige 31 est disposée dans la creusure 172 et le sabot 18 est disposé dans son dégagement 179. Les moitiés 111 et 112 du boîtier sont alors reliées au moyen des vis 12. On peut prévoir de disposer des caches sur les têtes des vis 12, afin d'éviter que le praticien ne les actionne inutilement.

Le dispositif est alors prêt à être utilisé. Au moment de sa mise en place, le dispositif est bloqué de la manière suivante :
- le manchon 32 est serré sur l'épaulement fileté 531,
- la tige intérieure 31 est bloquée angulairement dans le plan de l'appareil au moyen de la vis 13 appuyant sur le sabot 18,
- la vis 16 et le cavalier mobile 162 sont serrés pour éviter tout mouvement de la coulisse arquée 21,
- les écrous 25 et 26 sont serrés de part et d'autre de l'ergot 44, qui est disposé longitudinalement et angulairement dans une position moyenne.

Au cours de l'opération, le praticien insère les fiches 60 et 70 dans les os, de préférence avec un angle d'environ 45° par rapport au plan de la main, puis il met en place les ensembles 40 et 50 de fixation des fiches. Les aiguilles de visée 91 et 92 sont alors ajoutées et le réglage des brides 42 et 52 permet d'effectuer le positionnement du fixateur articulé selon les flèches A et B à la figure 1, pour que l'axe des pièces rotatives corresponde à l'axe de l'articulation. Ce réglage a lieu en contrôlant la position des os aux rayons X.

Les aiguilles de visée 91 et 92 sont enlevées, de même que la plaquette 93 dans le cas où l'on utilise la variante à plaquette amovible plutôt que des aiguilles passant dans des ouvertures du boîtier.

Le praticien desserre alors la vis 13 et l'un des écrous 25 ou 26, pour effectuer le réglage transversal selon la flèche C à la figure 2. En faisant faire des mouvements à la main du patient, après avoir déplacé temporairement la vis 16 et son cavalier 162 dans l'ouverture arquée 14, le praticien obtient la mise en place automatique du joint articulé dans le plans transversal, puis il bloque l'ensemble à l'aide de la vis 13 et des écrous 25 et 26. Il est à noter que ces dérniers permettent d'appliquer une légère tension au niveau de l'os fracturé, pour obtenir une décompression de la fracture.

Comme on l'a déjà indiqué, la mise en place est ainsi particulièrement simplifiée avec le type de fixateur décrit ici puisque le réglage dans le plan des flèches A et B de la figure 1 est indépendant du réglage transversal selon la flèche C à la figure 2. Avec les fixateurs connus précédemment, on devait agir au niveau des brides 42 et 52 de positionnement et on risquait par conséquent de dérégler l'ensemble lorsqu'une correction transversale était effectuée.

L'ensemble reste bloqué pendant environ 15 jours, pour avoir un début de consolidation osseuse. Ensuite on déplace progressivement la vis 16 et son cavalier 162, pour arriver en quelques semaines au mouvement de flexion maximum représenté à la figure 3.

En déplaçant les écrous 25 et 26 vers l'extérieur, on peut exercer un allongement des ligaments, pour faciliter la rééducation, en fin de traitement.

De plus, on peut desserrer progressivement le manchon 32 qui permet de donner de l'ébat au dispositif 30. Le praticien effectuera cette opération selon l'anatomie particulière de chaque patient afin que celui-ci puisse effectuer sans gêne le mouvement de flexion autorisé par le joint articulé.

## Revendications

1. Fixateur externe dynamique pour l'ostéosynthèse d'une articulation fracturée comprenant :
a) un premier membre (40) ayant une tige de fixation (43) apte à être reliée à des fiches (60) insérées dans l'os d'un côté de l'articulation (80);
b) un second membre (50) ayant une tige de fixation (53) apte à être reliée à des fiches (70) insérées dans un os de l'autre côté de l'articulation;
c) et un joint articulé (10) reliant les tiges (43,53) qui possède :
- un boîtier (11) présentant une surface de glissement courbe possédant un axe virtuel,
- des moyens de visée (90) dudit axe,
- un segment arqué (21) apte à glisser sur ladite surface,
caractérisé par le fait que les dites tiges (43,53) sont aptes à pivoter sur leur axe par rapport au boîtier (11), pour autoriser le positionnement transversal du joint articulé (selon la flèche C) sans affecter le réglage longitudinal (selon les flèches A et B).

2. Fixateur selon la revendication 1, caractérisé par le fait qu'une extrémité du segment arqué (21) est solidaire d'un corps cylindrique (23) muni d'une ouverture centrale extérieure (24) apte à recevoir l'extrémité de la tige (43), et muni de moyens de fixation de ladite tige.

3. Fixateur selon la revendication 2, caractérisé par le fait que l'ouverture centrale (24) comporte un dégagement latéral (27) et l'extrémité de la tige (43) présente un ergot radial (44) apte à être déplacé longitudinalement et radialement dans l'ouverture centrale (24) et son dégagement latéral (27).

4. Fixateur selon la revendication 2 ou 3, caractérisé par le fait que le corps cylindrique (23) est fileté et que les moyens de fixation de la tige sont constitués par deux écrous (25 et 26).

5. Fixateur selon la revendication 1, caractérisé par le fait que la tige (53) est solidaire d'un dispositif autorisant de l'ébat (30) muni de moyens de fixation au joint articulé (10).

6. Fixateur selon la revendication 5, caractérisé par le fait que le dispositif autorisant de l'ébat (30) comporte une tige intérieure (31) dont une extrémité est munie d'une tête (311) apte à maintenir l'ensemble dans un dégagement correspondant (171) du boîtier (11) du joint (10); et dont l'autre extrémité comporte un épaulement (313) et une ouverture centrale tronconique (315) apte à recevoir l'extrémité tronconique correspondante (532) de la tige (53), qui comporte un épaulement fileté (531) destiné à coopérer avec un manchon de serrage (32) apte à entourer le dispositif autorisant de l'ébat (30).

7. Fixateur selon la revendication 6, caractérisé par le fait que l'ouverture centrale (315) est prolongée latéralement par deux dégagements opposés (316) constituant une collerette de retenue (317) d'un axe (533) fixé transversalement à l'extrémité (532) de la tige (53).

8. Fixateur selon la revendication 6, caractérisé par le fait qu'un ressort de compression (34) est disposé entre les épaulements (313) de la tige intérieure (31) et (531) de la tige de fixation (53).

9. Fixateur selon la revendication 6, caractérisé par le fait que le boîtier (11) comporte des moyens de fixation (13) de la tige intérieure (31).

10. Fixateur selon la revendication 1, caractérisé par le fait que le boîtier (11) comporte au moins trois ensembles de roulement (191,192) aptes à coopérer avec le segment arqué (21), afin de remplacer le glissement du segment arqué par un roulement.

11. Fixateur selon la revendication 10, caractérisé par le fait que le segment arqué (21) présente en coupe deux épaulements (211,212) côniques opposés destinés à coopérer chacun avec une gorge (194) de forme correspondante d'un galet (193) apte à tourner par rapport à un arbre (197) fixé dans le boîtier (11).

12. Fixateur selon la revendication 11, caractérisé en ce que les extrémités de l'arbre (197) sont fixées dans des évidements (175 à 178) pratiqués dans les parois internes opposées du boîtier (11).

13. Fixateur selon la revendication 12, caractérisé par le fait que des paliers (198) entourés par un joint élastomère (199) sont insérés entre les extrémité de l'arbre (197) et les évidements (175 à 178).

14. Fixateur selon la revendication 11, caractérisé par le fait que des roulements à billes (196) sont disposés entre le galet (193) et l'arbre (197).

15. Fixateur selon la revendication 1, caractérisé par le fait que le segment arqué (21) comporte une butée transversale (22) en saillie dans une ouverture arquée (14) du boîtier (11).

16. Fixateur selon la revendication 15, caractérisé par le fait que des moyens de limitation réglables (16) sont disposés dans l'ouverture arquée (14).

17. Fixateur selon la revendication 1, caractérisé par le fait que le boîtier comporte des ouvertures de part en part aptes à racevoir des aiguilles amovibles (91,92) constituant les moyens de visée.

18. Fixateur selon la revendication 1, caractérisé par le fait que le boîtier comporte des moyens de fixation d'une plquette amovible (93) comportant des passages pour des aiguilles amovibles (91,92) constituant les moyens de visée.

## Claims

1. A dynamic external fixation device for osteosynthesis of a fractured articulation comprising:
a) a first member (40) having a fixation rod (43) capable of being connected to pins (60) inserted in the bone on one side of the articulation (80);
b) a second member (50) having a fixation rod (53) capable of being connected to pins (70) inserted in a bone on the other side of the articulation; and
c) an articulated joint (10) connecting the rods (43, 53), which has:
- a casing (11) having a curved sliding surface possessing a virtual axis,
- means (90) for targeting the said axis,
- an arcuate segment (21) sliding on the said surface,
characterised by the fact that the said rods (43, 53) are capable of pivoting on their axis relative to the casing (11) in order to permit the transverse positioning of the articulated joint (according to the arrow C) without affecting the longitudinal adjustment (according to the arrows A and B).

2. A fixation device according to claim 1, characterised by the fact that one end of said arc-shaped segment (21) is integral with a cylindrical body (23) which has an external central opening (24) capable of receiving an end of said first rod (43) and which has a means for fixation of said rod.

3. A fixation device according to claim 2, characterised by the fact that said central opening (24) comprises a lateral clearance (27) and in that an end of said rod (43) has a radial spur (44) capable of being displaced longitudinally and radially in said central opening (24) and its lateral clearance (27).

4. A fixation device according to claim 2 or 3, characterised by the fact that said cylindrical body (23) is threaded and wherein a means for fixation of said rod consists of two nuts (25, 26).

5. A fixation device according to claim 1, characterised by the fact that said second rod (53) is integral with a device which permits play (30) and which is provided with means for fixation to said articulating portion (10).

6. A fixation device according to claim 5, characterised by the fact that said device which permits play (30) comprises an inner rod (31) having one end thereof provided with a head (311) capable of maintaining the assembly in a corresponding clearance (171) in said casing (11) of said articulating portion (10) and having the other end thereof comprising a shoulder (313) and a truncated central opening (315) capable of receiving a corresponding truncated end (532) of said second rod (53), which comprises a threaded shoulder (531) intended to cooperate with a tightening sleeve (32) capable of surrounding said device which permits play (30).

7. A fixation device according to claim 6, characterised by the fact that said central opening (315) is extended laterally by two opposite clearances (316) constituting a retention collar (317) of an axis (533) which is fixed transversely to an end (532) of said second rod (53).

8. A fixation device according to claim 6, characterised by the fact that a compression spring (34) is arranged between the shoulder (313) of said inner rod (31) and the shoulder (531) of said second fixation rod (53).

9. A fixation device according to claim 6, characterised by the fact that said casing (11) comprises means for fixation (13) of said inner rod (31).

10. A fixation device according to claim 1, characterised by the fact that said casing (11) comprises at least two roller bearing assemblies (191, 192) capable of cooperating with said arc-shaped segment (21), in order to replace sliding action of said arc-shaped segment (21) by rolling action.

11. A fixation device according to claim 10, characterised by the fact that said arc-shaped segment (21) has in cross-section two opposite conical shoulders (211, 212) each intended to cooperate with a groove (194) of corresponding shape of a roller (193) capable of turning relative to a shaft (197) fixed in said casing (11).

12. A fixation device according to claim 11, characterised by the fact that the ends of said shaft (197) are fixed in recesses (175-178) formed in opposite inner walls of said casing (11).

13. A fixation device according to claim 12, characterised by the fact that bearings (198) surrounded by an elastomeric seal (199) are inserted between ends of said shaft (197) and said recesses (175-178).

14. A fixation device according to claim 11, characterised by the fact that ball-bearings (196) are arranged between said roller (193) and said shaft (197).

15. A fixation device according to claim 1, characterised by the fact that said arc-shaped segment (21) comprises a transverse stop (22) which projects into an arc-shaped opening (14) in said casing (11).

16. A fixation device according to claim 15, characterised by the fact that an adjustable limitation means (16) is arranged in said arc-shaped opening (14).

17. A fixation device according to claim 1, characterised by the fact that said casing comprises openings capable of receiving removable targeting means (91, 92).

18. A fixation device according to claim 1, characterised by the fact that casing comprises means for fixation of a removable plate (93) which comprises passages for removable targeting means (91, 92).

## Patentansprüche

1. Dynamische Außenbefestigung für die Osteosynthese eines gebrochenen Gelenkes, die folgendes aufweist:
a) ein erstes Teil (40) mit einem Befestigungsschaft (43), welcher an Steckverbindungen (60) angeschlossen werden kann, die auf einer Seite des Gelenkes (80) eingeschoben werden;
b) ein zweites Teil (50) mit einem Befestigungsschaft (53), welcher an die Steckverbindungen (70) angeschlossen werden kann, die auf der anderen Seite des Gelenkes in den Knochen eingeschoben werden;
c) und ein die Schäfte (43, 53) verbindendes Universalgelenk (10), welches folgendes aufweist:
- ein Gehäuse (11) mit einer bogenförmigen Gleitführung mit einer virtuellen Achse,
- Visurmittel (90) für diese Achse,
- ein bogenförmiges Segment (21), welches auf dieser Fläche gleiten kann,
**dadurch gekennzeichnet**, **daß**
diese Schäfte (43, 53) gegenüber dem Gehäuse (11) um ihre Achse geschwenkt werden können. um auf diese Weise die Positionierung des Universalgelenks in Querrichtung (entlang dem Pfeil C) zu ermöglichen, ohne die Regulierung in Längsrichtung (in Richtung der Pfeile A und B) zu verändern.

2. Befestigung nach Anspruch 1,
**dadurch gekennzeichnet**, **daß**
ein Endteil des bogenförmigen Segmentes (21) fest mit einem zylindrischen Körper (23) verbunden ist, welcher eine äußere zentrale Öffnung (24) besitzt, die den Endteil des Schaftes (43) aufnimmt, und mit Mitteln für die Befestigung der Schäfte versehen ist.

3. Befestigung nach Anspruch 2,
**dadurch gekennzeichnet**, **daß**
die zentrale Öffnung (24) einen seitlichen Einstich (27) aufweist und der Endteil des Schaftes (43) mit einer radialen Nase (44) versehen ist, die in Längsrichtung und radial in der zentralen Öffnung (24) und ihrem seitlichen Einstich (27) bewegt werden kann.

4. Befestigung nach einem der Ansprüche 2 oder 3,
**dadurch gekennzeichnet**, **daß**
der zylindrische Körper (23) mit einem Gewinde versehen ist, und daß die Mittel für die Befestigung der Schäfte aus zwei Muttern (25 und 26) bestehen.

5. Befestigung nach Anspruch 1,
**dadurch gekennzeichnet**, **daß**
der Schaft (53) mit einer ein Längsspiel ermöglichenden Vorrichtung (30) verbunden ist, welche mit Mitteln für die Befestigung des Universalgelenks (10) ausgerüstet ist.

6. Befestigung nach Anspruch 5,
**dadurch gekennzeichnet**, **daß**
die das Längsspiel ermöglichende Vorrichtung (30) einen inneren Schaft (31) aufweist, bei dem ein Ende mit einem Kopfteil (311) ausgestattet ist, welcher das Bauteil in einem entsprechenden Freistich (171) des Gehäuses (11) des Universalgelenkes (10) hält, während das andere Ende eine Schulter (313) und eine stumpfkegelige zentrale Öffnung (315) aufweist, welche den entsprechenden stumpfkegeligen Endteil (532) des Schaftes (53) aufnimmt, welcher einen Gewindeansatz (531) enthält, der dafür bestimmt ist, mit einer Gewindemanschette (32) zusammenzuwirken, welche die das Längsspiel ermöglichende Vorrichtung (30) umhüllt.

7. Befestigung nach Anspruch 6,
**dadurch gekennzeichnet**, **daß**
die zentrale Öffnung (315) seitlich durch zwei gegenüberliegende Freistiche (316) verlängert wird, welche einen Halterungskragen (317) für eine Welle (533) bilden, der quer zu dem Endteil (532) des Schaftes (53) angeordnet ist.

8. Befestigung nach Anspruch 6,
**dadurch gekennzeichnet**, **daß**
eine Druckfeder (34) zwischen den Schulteransätzen (313) des inneren Schäfte (31) und (531) des Befestigungsschaftes (53) angeordnet ist.

9. Befestigung nach Anspruch 6,
**dadurch gekennzeichnet**, **daß**
das Gehäuse (11) Mittel (13) für die Befestigung des inneren Schaftes (31) aufweist.

10. Befestigung nach Anspruch 1,
**dadurch gekennzeichnet**, **daß**
das Gehäuse (11) mindestens drei Kugellager (191, 192) enthält, welche mit dem bogenförmigen Segment (21) zusammenwirken können, um auf diese Weise die Gleitbewegung des bogenförmigen Segmentes durch eine Rollbewegung zu ersetzen.

11. Befestigung nach Anspruch 10,
**dadurch gekennzeichnet**, **daß**
das bogenförmige Segment (21) im Querschnitt zwei gegenüberliegende konische Schulteransätze (211, 212) aufweist, welche jeweils mit einer Nut (194) entsprechender Form eines Wälzkörpers (193) zusammenwirken können, welcher sich gegenüber einer in dem Gehäuse (11) befestigten Welle (197) dreht.

12. Befestigung nach Anspruch 10,
**dadurch gekennzeichnet**, **daß**
die Enden der Welle (197) in den Freistichen (175 bis 178) befestigt sind, welche in den gegenüberliegenden inneren Wände des Gehäuses (11) eingebracht sind.

13. Befestigung nach Anspruch 12,
**dadurch gekennzeichnet**, **daß**
das von einer Dichtung (199) aus einem Elastomerwerkstoff umgebene Lager (198) zwischen dem Ende der Welle (197) und den Freistichen (175 bis 178) eingesetzt wird.

14. Befestigung nach Anspruch 11,
**dadurch gekennzeichnet**, **daß**
die Kugellager (196) zwischen dem Wälzkörper (193) und der Welle (197) angeordnet sind.

15. Befestigung nach Anspruch 1,
**dadurch gekennzeichnet**, **daß**
das bogenförmige Segment (21) einen schrägen Anschlag (22) aufweist, welcher aus einer bogenförmigen Öffnung (14) des Gehäuses (11) herausragt.

16. Befestigung nach Anspruch 15,
**dadurch gekennzeichnet**, **daß**
in der bogenförmigen Öffnung (14) regulierbare Begrenzungsmittel (16) angeordnet sind.

17. Befestigung nach Anspruch 1,
**dadurch gekennzeichnet**, **daß**
das Gehäuse auf beiden Seiten Öffnungen aufweist, welche die die Visurmittel bildenden löslichen Zeiger (91, 92) aufnehmen können.

18. Befestigung nach Anspruch 1,
**dadurch gekennzeichnet**, **daß**
das Gehäuse Mittel für die Befestigung einer löslichen Federscheibe (93) aufweist, welche Durchgänge für die die Visurmittel bildenden Zeiger (91, 92) enthält.
